# FASCICULE DE BREVET EUROPEEN

(11) **EP 3 204 492 B1**
(45) Date de publication et mention de la délivrance du brevet: **09.03.2022**
(21) Numéro de dépôt: 15771979.0
(22) Date de dépôt: 05.10.2015
(51) Int. Cl.: C12N 5/0783, A61P 31/00, A61P 37/00

(54) **MÉTHODE DE GÉNÉRATION DE PROGÉNITEURS DE CELLULES T**
VERFAHREN ZUR ERZEUGUNG VON T-ZELL-VORLÄUFERN
METHOD FOR GENERATING T-CELL PROGENITORS

(30) Priorité: 06.10.2014 FR 1459539
(43) Date de publication de la demande: 16.08.2017
(60) Demande divisionnaire: 22152734.4
(73) Titulaire: Assistance Publique-Hôpitaux de Paris, 75004 Paris (FR); Fondation Imagine - Institut des Maladies Génétiques, 75015 Paris (FR); Université de Paris, 75006 Paris (FR); Institut National de la Santé et de la Recherche Médicale (INSERM), 75013 Paris (FR)
(72) Inventeur: CAVAZZANA, Marina, 75015 Paris (FR); ANDRÉ, Isabelle, 92190 Meudon (FR); LAGRESLE-PEYROU, Chantal, 92120 Montrouge (FR); HACEIN-BEY-ABINA, Salima, 92250 La Garenne Colombes (FR); REIMANN, Christian, 75015 Paris (FR); DE CHAPPEDELAINE, Corinne, 75015 Paris (FR); SIX, Emmanuelle, 91700 Saint Geneviève des Bois (FR)
(74) Mandataire: Icosa
(86) Numéro de dépôt international: PCT/EP2015/072889
(87) Numéro de publication internationale: WO 2016/055396

(56) Documents cités:
- WO-A1-2011/068962
- WO-A1-2014/110353
- CHRISTIAN REIMANN ET AL: "Human T-Lymphoid Progenitors Generated in a Feeder-Cell-Free Delta-Like-4 Culture System Promote T-Cell Reconstitution in NOD/SCID/[gamma]c-/- Mice", STEM CELLS, vol. 30, no. 8, 24 août 2012 (2012-08-24), pages 1771-1780, XP055192943, ISSN: 1066-5099, DOI: 10.1002/stem.1145
- Kim and Roy: "Ligand-functionalized biomaterial surfaces: controlled regulation of signaling pathways to direct stem cell differentiation" In: "Biological interactions on materials surfaces", 2009, Puleo and Bizios, XP009184615, pages 157-171, le document en entier § 8.2.3; page 164 - page 166 § 8.3.2; page 168

### SEQUENCE LISTING

<110> ASSISTANCE PUBLIQUE HÔPITAUX DE PARIS FONDATION IMAGINE - INSTITUT DES MALADIES GÉNÉTIQUES UNIVERSITÉ PARIS DESCARTES INSTITUT NATIONAL DE LA SANTÉ ET DE LA RECHERCHE MÉDICALE (INSERM)
<120> Méthode de génération de progéniteurs de cellules T
<130> BRV 79 - WO
<150> FR 14/59539
   <151> 2014-10-06
<160> 7
<170> PatentIn version 3.3
<210> 1
   <211> 723
   <212> PRT
   <213> artificial
<220>
   <223> Delta -1 (soluble: 1-536) G502 peut être R
<400> 1
<210> 2
   <211> 724
   <212> PRT
   <213> artificial
<220>
   <223> Delta-4 (fraction soluble 1-526)
<400> 2
<210> 3
   <211> 4
   <212> PRT
   <213> artificial
<220>
   <223> Motif RGDS
<400> 3
   Arg Gly Asp Ser
   1
<210> 4
   <211> 6
   <212> PRT
   <213> artificial
<220>
   <223> B = acide aminé basique; X = acide aminé hydropathique
<220>
   <221> misc_feature
   <222> (1)..(1)
   <223> Xaa can be any naturally occurring amino acid
<220>
   <221> misc_feature
   <222> (4)..(4)
   <223> Xaa can be any naturally occurring amino acid
<220>
   <221> misc_feature
   <222> (6)..(6)
   <223> Xaa can be any naturally occurring amino acid
<400> 4
   Xaa Asx Asx Xaa Asx Xaa
   1               5
<210> 5
   <211> 8
   <212> PRT
   <213> artificial
<220>
   <223> B = acide aminé basique; X = acide aminé hydropathique
<220>
   <221> misc_feature
   <222> (1)..(1)
   <223> Xaa can be any naturally occurring amino acid
<220>
   <221> misc_feature
   <222> (5)..(6)
   <223> Xaa can be any naturally occurring amino acid
<220>
   <221> misc_feature
   <222> (8)..(8)
   <223> Xaa can be any naturally occurring amino acid
<400> 5
   Xaa Asx Asx Asx Xaa Xaa Asx Xaa
   1               5
<210> 6
   <211> 25
   <212> PRT
   <213> artificial
<220>
   <223> Motif CS-1
<400> 6
   Asp Glu Leu Pro Gln Leu Val Thr Leu Pro His Pro Asn Leu His Gly
   1               5                   10                  15
   Pro Glu Ile Leu Asp Val Pro Ser Thr
               20                  25
<210> 7
   <211> 754
   <212> **PRT**
   <213> artificial
<220>
   <223> Portéine de fusion delta 4 - Fc
<400> 7
   Met Ala Ala Ala Ser Arg Ser Ala Ser Gly Trp Ala Leu Leu Leu Leu
   1               5                   10                  15
   Val Ala Leu Trp Gln Gln Arg Ala Ala Gly Ser Gly Val Phe Gln Leu
               20                  25                  30
   Gln Leu Gln Glu Phe Ile Asn Glu Arg Gly Val Leu Ala Ser Gly Arg
           35                  40                  45
   Pro Cys Glu Pro Gly Cys Arg Thr Phe Phe Arg Val Cys Leu Lys His
       50                  55                  60
   Phe Gln Ala Val Val Ser Pro Gly Pro Cys Thr Phe Gly Thr Val Ser
   65                  70                  75                  80
   Thr Pro Val Leu Gly Thr Asn Ser Phe Ala Val Arg Asp Asp Ser Ser
                   85                  90                  95
   Gly Gly Gly Arg Asn Pro Leu Gln Leu Pro Phe Asn Phe Thr Trp Pro
               100                 105                 110
   Gly Thr Phe Ser Leu Ile Ile Glu Ala Trp His Ala Pro Gly Asp Asp
           115                 120                 125
   Leu Arg Pro Glu Ala Leu Pro Pro Asp Ala Leu Ile Ser Lys Ile Ala
       130                 135                 140
   Ile Gln Gly Ser Leu Ala Val Gly Gln Asn Trp Leu Leu Asp Glu Gln
   145                 150                 155                 160
   Thr Ser Thr Leu Thr Arg Leu Arg Tyr Ser Tyr Arg Val Ile Cys Ser
                   165                 170                 175
   Asp Asn Tyr Tyr Gly Asp Asn Cys Ser Arg Leu Cys Lys Lys Arg Asn
               180                 185                 190
   Asp His Phe Gly His Tyr Val Cys Gln Pro Asp Gly Asn Leu Ser Cys
           195                 200                 205
   Leu Pro Gly Trp Thr Gly Glu Tyr Cys Gln Gln Pro Ile Cys Leu Ser
       210                 215                 220
   Gly Cys His Glu Gln Asn Gly Tyr Cys Ser Lys Pro Ala Glu Cys Leu
   225                 230                 235                 240
   Cys Arg Pro Gly Trp Gln Gly Arg Leu Cys Asn Glu Cys Ile Pro His
   245 250 255
   Asn Gly Cys Arg His Gly Thr Cys Ser Thr Pro Trp Gln Cys Thr Cys
   260 265 270
   Asp Glu Gly Trp Gly Gly Leu Phe Cys Asp Gln Asp Leu Asn Tyr Cys
   275 280 285
   Thr His His Ser Pro Cys Lys Asn Gly Ala Thr Cys Ser Asn Ser Gly
   290 295 300
   Gln Arg Ser Tyr Thr Cys Thr Cys Arg Pro Gly Tyr Thr Gly Val Asp
   305 310 315 320
   Cys Glu Leu Glu Leu Ser Glu Cys Asp Ser Asn Pro Cys Arg Asn Gly
   325 330 335
   Gly Ser Cys Lys Asp Gln Glu Asp Gly Tyr His Cys Leu Cys Pro Pro
   340 345 350
   Gly Tyr Tyr Gly Leu His Cys Glu His Ser Thr Leu Ser Cys Ala Asp
   355 360 365
   Ser Pro Cys Phe Asn Gly Gly Ser Cys Arg Glu Arg Asn Gln Gly Ala
   370 375 380
   Asn Tyr Ala Cys Glu Cys Pro Pro Asn Phe Thr Gly Ser Asn Cys Glu
   385 390 395 400
   Lys Lys Val Asp Arg Cys Thr Ser Asn Pro Cys Ala Asn Gly Gly Gln
   405 410 415
   Cys Leu Asn Arg Gly Pro Ser Arg Met Cys Arg Cys Arg Pro Gly Phe
   420 425 430
   Thr Gly Thr Tyr Cys Glu Leu His Val Ser Asp Cys Ala Arg Asn Pro
   435 440 445
   Cys Ala His Gly Gly Thr Cys His Asp Leu Glu Asn Gly Leu Met Cys
   450 455 460
   Thr Cys Pro Ala Gly Phe Ser Gly Arg Arg Cys Glu Val Arg Thr Ser
   465 470 475 480
   Ile Asp Ala Cys Ala Ser Ser Pro Cys Phe Asn Arg Ala Thr Cys Tyr
                   485                 490                 495
   Thr Asp Leu Ser Thr Asp Thr Phe Val Cys Asn Cys Pro Tyr Gly Phe
               500                 505                 510
   Val Gly Ser Arg Cys Glu Phe Pro Val Gly Leu Pro Pro Ser Thr Met
           515                 520                 525
   Val Arg Ser Val Glu Cys Pro Pro Cys Pro Ala Pro Pro Val Ala Gly
       530                 535                 540
   Pro Ser Val Phe Leu Phe Pro Pro Lys Pro Lys Asp Thr Leu Met Ile
   545                 550                 555                 560
   Ser Arg Thr Pro Glu Val Thr Cys Val Val Val Asp Val Ser His Glu
                   565                 570                 575
   Asp Pro Glu Val Gln Phe Asn Trp Tyr Val Asp Gly Met Glu Val His
               580                 585                 590
   Asn Ala Lys Thr Lys Pro Arg Glu Glu Gln Phe Asn Ser Thr Phe Arg
           595                 600                 605
   Val Val Ser Val Leu Thr Val Val His Gln Asp Trp Leu Asn Gly Lys
       610                 615                 620
   Glu Tyr Lys Cys Lys Val Ser Asn Lys Gly Leu Pro Ala Pro Ile Glu
   625                 630                 635                 640
   Lys Thr Ile Ser Lys Thr Lys Gly Gln Pro Arg Glu Pro Gln Val Tyr
                   645                 650                 655
   Thr Leu Pro Pro Ser Arg Glu Glu Met Thr Lys Asn Gln Val Ser Leu
               660                 665                 670
   Thr Cys Leu Val Lys Gly Phe Tyr Pro Ser Asp Ile Ala Val Glu Trp
           675                 680                 685
   Glu Ser Asn Gly Gln Pro Glu Asn Asn Tyr Lys Thr Thr Pro Pro Met
       690                 695                 700
   Leu Asp Ser Asp Gly Ser Phe Phe Leu Tyr Ser Lys Leu Thr Val Asp
   705                 710                 715                 720
   Lys Ser Arg Trp Gln Gln Gly Asn Val Phe Ser Cys Ser Val Met His
                   725                 730                 735
   Glu Ala Leu His Asn His Tyr Thr Gln Lys Ser Leu Ser Leu Ser Pro
               740                 745                 750
   Gly Lys

## Description

L'invention se rapporte au domaine de la thérapie cellulaire et en particulier des greffes de cellules souches hématopoïétiques, transformées ou non, et à la reconstitution immunitaire à la suite de telles greffes.

La greffe de Progéniteurs et Cellules Souches Hématopoïétiques (HSPC pour Hematopoietic Stem and Progenitor Cells) est considérée comme la meilleure option thérapeutique pour les déficits immunitaires héréditaires les plus sévères, pour de nombreuses hémopathies malignes, ainsi que pour un certain nombre de tumeurs solides.

Actuellement, dans les situations d'allogreffes avec incompatibilité HLA partielle, l'injection aux receveurs préalablement conditionnés de doses croissantes de HSPC CD34+ triées permet la prise de greffe du donneur avec une prévention efficace de la maladie du greffon contre l'hôte (GVHD). Néanmoins, la différenciation de nouveaux lymphocytes T à partir des cellules CD34+ injectées, nécessite une période minimale de 4 mois et ces lymphocytes T ne sont en nombre suffisant pour jouer un rôle protecteur vis à vis des infections que quelques mois après leur apparition.

C'est à cette lenteur de la reconstitution immunitaire que l'on doit les nombreuses complications infectieuses notamment virales, mais également les rechutes, qui influent sur le pronostic à long terme des patients greffés.

Par ailleurs, d'autres protocoles thérapeutiques utilisent une approche de thérapie génique, c'est-à-dire une autogreffe de HPSC transduites, qui a démontré son efficacité dans le traitement de certains déficits immunitaires héréditaires. L'intérêt de cette stratégie par rapport à la greffe de HSPC CD34+ allogéniques est indiscutable en termes de survie et de morbidité quand aucun donneur HLA-compatible n'est disponible. Néanmoins, l'expérience clinique a montré que pour certains patients présentant des infections sévères, la reconstitution du compartiment lymphocytaire T est lente et n'atteint jamais des niveaux normaux de lymphocytes T circulants. Les morbidité et mortalité associées à ce contexte particulier sont importantes.

De par le nombre de patients concernés, de par l'importante morbidité et mortalité qui suit ce type de greffe, la mise au point de nouvelles thérapeutiques visant à diminuer la période d'immunodéficience suivant la greffe est pleinement justifiée.

Il est donc important d'accélérer la génération des lymphocytes T par l'administration de précurseurs engagés dans la voie de différenciation lymphoïde T (progéniteurs de cellules T).

Ces précurseurs de cellules T sont issus d'une différenciation de HSPC CD34+ et présentent notamment le marqueur CD7+, qui est un marqueur de différenciation dans la voie des lymphocytes T. Ils peuvent également présenter d'autres marqueurs. Ainsi, Awong et al. (Blood 2009;114:972-982) ont décrit les précurseurs suivants des lymphocytes T : les progéniteurs précoces thymiques (early thymic progenitor (ETP)), qui présentent les marqueurs (CD34+/CD45RA+/CD7+), les cellules au stade proT1 (CD7++/CD5-), les cellules au stade proT2 (CD7++/CD5+), les cellules au stade préT (CD7++/CD5+CD1a+). Les HSPC acquièrent donc ces marqueurs de façon successive en passant par ces étapes, lors du développement de cellules T.

Une greffe de précurseurs T concomitante à la greffe de HSPC permettrait la production rapide d'un compartiment lymphocytaire T mature et fonctionnel, et préviendrait de ce fait, le risque d'infections sévères, en permettant au patient de bénéficier d'un niveau d'immunité minimal avant la reconstitution totale de son système immunitaire.

Par ailleurs, il est important de pouvoir utiliser des cellules adultes plutôt que des cellules de sang de cordon, dans la mesure où il est plus facile et moins onéreux d'obtenir des cellules adultes, que des cellules de sang de cordon et qu'elles sont plus utilisées en allogreffe.

Toutefois, les données publiées dans la littérature, obtenues chez l'homme et la souris, montrent des différences intrinsèques entre cellules hématopoïétiques fœtales (incluant le sang de cordon) et cellules adultes. Ces différences portent sur la survie, la capacité à réparer les dommages à l'ADN, la capacité proliférative et le potentiel de différenciation (voir notamment Yuan et al. (Science. 2012 Mar 9;335(6073):1195-200), qui indiquent que les cellules de moelle osseuse adultes sont moins efficaces que les cellules fœtales dans leur potentiel à générer divers types cellulaires; Lansdorp et al. (J Exp Med. 1993 Sep 1;178(3):787-91) ; Szilvassy et al. (Blood. 2001 Oct 1;98(7):2108-15); Frassoni et al. (Blood. 2003 Aug 1;102(3):1138-41); Liang et al. (Blood. 2005 Aug 15;106(4):1479-87) ; Six et al. (J Exp Med. 2007 Dec 24;204(13):3085-93)).

Les protéines Notch sont des récepteurs transmembranaires qui régulent la réponse cellulaire à un grand nombre de signaux environnementaux. Chez les mammifères, 4 récepteurs Notch (Notch 1-4) et cinq ligands (Delta-like-1, 3, et 4, jagged1, jagged2) ont été décrits (Weinmaster Curr Opin Genet Dev. 2000;10:363-369).

Les ligands de Notch ont plusieurs dénominations. Ainsi, le ligand Delta-like-ligand 4 peut être désigné comme :
- Delta-like-ligand 4 (correspondant au nom du gène DLL4)
- ou par simplification Delta-like-4 ou Delta ligand 4 (abréviation DL-4).

Dans la présente demande, les ligands de Notch Delta-like-1 et Delta-like-4 pourront être désignés respectivement par DL1 et DL4 ou par DL-1 et DL-4. Les séquences des ligands DL-1 et DL-4 sont précisées en tant que SEQ ID N° 1 et SEQ ID N° 2, respectivement.

Il a été montré que l'interaction de Notch-1 avec les ligands DL-1 ou DL-4 joue un rôle important dans la lymphopoïèse T précoce.

Ainsi, plusieurs groupes de recherche ont réussi à induire une différenciation T murine et humaine *in vitro* par coculture de progéniteurs hématopoïétiques, par exemple avec des cellules stromales OP9 ou Tst-4 transduites avec le ligand DL-1 ou le ligand DL-4. Il a ainsi été montré que les précurseurs ainsi générés permettaient la production de lymphocytes T matures après greffe à des souris immunodéficientes irradiées (voir notamment Zakrzewski et al., Nat Med 2006 12(9): 1039 et Nat Biotech 2008 26(4): 453; Awong et al., Blood 2009 114 (5) :972; Meek et al., Blood 2010. 115:261-264).

Toutefois, l'utilisation d'un stroma murin génétiquement modifié ne peut être envisagée dans un contexte clinique.

Il s'agit de développer un procédé permettant de générer et augmenter le nombre de précurseurs lymphocytaires T CD7+, à partir de cellules souches hématopoïétiques CD34+ sans utiliser de stroma cellulaire. De préférence, les cellules souches hématopoïétiques ont été isolées à partir d'un donneur adulte.

Dans l'espoir de remplacer le stroma OP9/DL1 par un système cliniquement plus acceptable, les inventeurs ont testé différents ligands de Notch solubles, et démontré qu'une forme soluble du ligand DL-4, fusionnée avec un fragment Fc d'une immunoglobuline (DL4/Fc), permettait de générer des cellules CD7+ à partir de progéniteurs CD34+ de sang de cordon (Reimann et al., Stem Cells 2012 ; 30 :1771-1780).

Ces travaux ont montré que 7 jours d'exposition au ligand DL4/Fc permettent d'obtenir un grand nombre de progéniteurs T, CD7+CD34+/-. L'analyse quantitative par Taqman-PCR des populations générées pour l'expression des gènes cibles de Notch et des gènes impliqués dans la différenciation des lymphocytes T, a montré que les changements phénotypiques correspondaient à un engagement dans la voie de différenciation lymphoïde T, en particulier avec une hausse de pTa, Rag1, IL7Rα et BCL11b, et une baisse de l'expression de facteurs de transcription impliqués dans les voies de différenciation myéloïde et lymphoïde B (notamment Pax5). Ainsi, les populations générées sur DL4/Fc correspondaient phénotypiquement et moléculairement aux populations retrouvées dans le thymus humain.

Ces travaux ont également montré l'absence de réarrangements des *loci* du récepteur de cellules T. Le potentiel lymphoïde T des progéniteurs générés en culture a été évalué *in vitro* et *in vivo. In vitro,* la quantification du potentiel lymphoïde T en condition de dilutions limites dans des cultures secondaires sur un stroma OP9 exprimant le ligand DL-1 a démontré une augmentation de plus de 200 fois par rapport aux HPSC CD34+ non cultivées. *In vivo,* ce potentiel a été déterminé par injection de ces cellules dans des souris NOD/SCID/γc-/- irradiées de 4 semaines ou nouveau-nés. Une thymopoïèse active a été retrouvée dans 20/22 souris ayant reçu des cellules cultivées sur DL4/Fc, contre 13/19 des souris ayant reçu des cellules CD34+ non cultivées. Des lymphocytes T matures ont été détectés uniquement dans les receveurs de cellules cultivées sur DL4/Fc. Les études de répertoire et les tests fonctionnels ont démontré que les lymphocytes T générés à partir des précurseurs T DL4/Fc présentaient un répertoire polyclonal et étaient fonctionnels.

Ces résultats montrent que l'exposition des cellules CD34+ de sang de cordon à DL4/Fc permet l'induction du développement lymphoïde T et la génération d'un grand nombre de précurseurs T *in vitro.* En outre, les précurseurs générés promeuvent une thymopoïèse humaine *in vivo* après greffe de souris immunodéficientes irradiées. Ainsi, alors que les publications antérieures ne faisaient état que d'une thymopoïèse active, des cellules T humaines matures circulent en périphérie chez ces souris très précocement après la greffe.

Ce système présente toutefois un certain nombre de limites :
1) D'un point de vue quantitatif, le système DL4/Fc permet d'obtenir environ 6.5×10⁵ progéniteurs T à partir de 10⁶ CD34+ de sang de cordon. Ce nombre pourrait se révéler insuffisant pour un adulte recevant une greffe de sang de cordon.
2) La molécule DL4/Fc est immobilisée sur le plastique des boites de culture. Cette étape d'immobilisation est importante pour permettre l'engagement des HPSC dans la voie lymphoïde T. Mais ce système présente trois limites, en termes de :
   - efficacité de l'immobilisation
   - demi-vie de la molécule immobilisée
   - liaison de DL4/Fc à son récepteur Notch à la surface des HSPC CD34+

Delaney et al. (Nat Med. 2010 February ; 16(2): 232) ont décrit des cultures de cellules souches de sang de cordon en présence d'un ligand de Notch (DL-1). L'objectif était d'accélérer la reconstitution hématopoïétique après greffe. Il convient toutefois de noter que les auteurs parlent d'augmenter le nombre de « progéniteurs » et que ce terme reflète en fait les cellules souches et les progéniteurs multipotents hématopoïétiques, en particulier myéloïdes. Les cellules ont été cultivées pendant 17-21 jours.

Ohishi et al. (J Clin Invest. 2002 Oct;110(8):1165-74), cité dans Delaney *et al.* décrivent également une culture de cellules souches issues de sang de cordon *in vitro* pour augmenter la capacité de repopulation du thymus et de la moelle osseuse lors d'une greffe. Les cellules sont également cultivées pendant 17 ou 18 jours.

Ces auteurs ne décrivent toutefois pas l'utilisation de cellules souches issues de donneurs adultes, ni le fait de cultiver ces cellules pour les utiliser pour reconstituer une population de précurseurs de lymphocytes T permettant de fournir une protection immunitaire à un receveur, dans les semaines suivant une greffe de moelle osseuse.

Kim et Roy (2009, In: "Biological interactions on materials surfaces", 2009, Puleo and Bizios, pages 157-171) exposent l'utilisation de biomatériaux présentant des ligands immobilisés pour contrôler et orienter la différenciation de cellules souches. La culture de cellules souches hématopoïétiques en présence de microbilles présentant le ligand Delta-like-4 de Notch aide à leur différenciation en lymphocytes T. La fibronectine immobilisée sur le support cellulaire améliore l'expansion des cellules souches. Des résultats similaires sont obtenus avec des peptides immobilisés comprenant par exemple les motifs RGDS et CS-1.

WO 2011/068962 décrit des procédés de différenciation de lymphocytes T ou NK *in vitro* à partir de cellules souches hématopoïétiques ou de cellules précurseurs. La première étape du procédé de différenciation de lymphocytes T permet la production de progéniteurs de lymphocytes T en cultivant les cellules souches sur une couche de cellules nourricières OP-9 transformées exprimant le ligand de Notch DL1. Le premier exemple illustre la génération de progéniteurs de lymphocytes T à partir de cellules souches murines de moelle osseuse.

WO 2014/110353 concerne des compositions comprenant un ligand de Notch ostéo-inducteur lié à au moins un substrat biocompatible. Elle concerne également des procédés de traitement de patients nécessitant une formation de tissu osseux par administration d'une composition comprenant un ligand de Notch ostéo-inducteur lié à au moins un substrat biocompatible.

Huijskens et al. (2014, Journal of Leukocyte Biology, 96(6) :1165-75) exposent un procédé pour obtenir en 3 semaines des cellules CD7+ à partir de cellules CD34+ de sang de cordon, cultivées sans système nourricier, en présence du ligand de Notch DLL4 :Fc et de Retronectin^{®} immobilisés sur la plaque de culture.

Il est donc nécessaire de développer une nouvelle méthode permettant d'améliorer la quantité de précurseurs de lymphocytes T générés à partir de cellules CD34+. Par ailleurs, cette méthode devrait également permettre l'obtention de précurseurs de lymphocytes T, y compris à partir des HSPC CD34+ transduites, qui puisse être applicable sur des cellules CD34+ adultes pour des greffes allogéniques ou des autogreffes, permettant ainsi de palier le nombre relativement faible de cellules de sang de cordon.

Les inventeurs ont montré que l'on pouvait améliorer la quantité de précurseurs de cellules T à partir de cellules CD34+ en exposant lesdites cellules CD34+ à un ligand de Notch immobilisé sur un support, en présence d'un fragment de fibronectine, contenant un motif RDGS (SEQ ID N° 3, Arginine - Glycine - Aspartate - Sérine), et/ou un motif CS-1 et éventuellement un domaine de liaison à l'héparine. De préférence, ledit fragment de fibronectine, contient un motif RDGS, un motif CS-1 et un domaine de liaison à l'héparine.

La présente invention concerne donc un procédé *in vitro* de génération de progéniteurs de cellules T, comprenant l'étape d'exposer des cellules CD34+ dans un milieu en présence d'un ligand de Notch, immobilisé sur un support, ledit ligand de Notch étant le domaine soluble du ligand Delta-like-4, fusionné à une région Fc d'une protéine IgG, et en présence d'un fragment d'une fibronectine, ledit fragment comprenant les motifs RGDS, CS-1 et un domaine de liaison à l'héparine, dans lequel le temps d'exposition des cellules CD34+ audit ligand de Notch en présence dudit fragment d'une fibronectine est inférieur à 10 jours, de préférence compris entre 3 et 8 jours.

Cette exposition conjointe des cellules CD34+ au ligand de Notch et à ce fragment de fibronectine permet également d'induire la différenciation de cellules CD34+ transduites vers la voie lymphoïde T, ce qui permet l'utilisation du procédé pour des greffes en thérapie génique.

On pense que l'effet observé est dû à la présence du motif RGDS et/ou du motif CS-1 dans le fragment de fibronectine. Ainsi, les procédés et méthodes décrits ci-dessous sont également applicables en utilisant un peptide RGDS et/ou un peptide CS-1 en place du fragment de fibronectine. Une utilisation conjointe des peptides RGDS et CS-1 est toutefois préférée, en particulier au sein de la même protéine.

Si l'on utilise un peptide RGDS et/ou un peptide CS-1, ils peuvent ainsi être présents en tant que tels au sein du milieu de culture, ou présents au sein d'un peptide ou d'une protéine présente dans ledit milieu. Lorsque le milieu de culture ne contient que le peptide RGDS et/ou le motif CS-1 en tant que tels, on parlera de peptide « libre » dans ledit milieu si ce peptide n'est pas immobilisé sur la paroi inférieure du récipient. En effet, le peptide peut être présent en solution, ou immobilisé sur la paroi inférieure du récipient dans lequel l'exposition des cellules CD34+ au ligand de Notch est réalisée.

Dans le mode de réalisation préféré, l'invention se rapporte à une méthode (ou un procédé) de génération de progéniteurs de cellules T, comprenant l'étape d'exposer des cellules CD34+ dans un milieu en présence d'un ligand de Notch, en présence d'un fragment de fibronectine. Le ligand de Notch est immobilisé sur un support. Le fragment de fibronectine contient un motif RDGS, un motif CS-1 et un domaine de liaison à l'héparine.

Le procédé ainsi décrit est réalisé *in vitro,* dans un récipient (tel qu'une boîte de culture cellulaire (boîte de Pétri, boîte 24 puits...)) sur la paroi inférieure duquel est immobilisé le ligand de Notch. Le ligand de Notch peut aussi être immobilisé sur tout autre support présent dans le milieu de réaction, en particulier à la surface de microbilles. L'immobilisation du ligand de Notch vise essentiellement à stabiliser le ligand pour permettre l'activation du récepteur Notch.

Par « progéniteur de cellules T », on entend désigner toute cellule engagée dans la voie de différenciation vers la voie lymphoïde T à partir d'une HSPC CD34+. Cette cellule est donc caractérisée en ce qu'elle exprime le marqueur CD7, qui est connu comme étant un des marqueurs apparaissant le plus précocement pendant la lymphopoïèse des cellules T. Selon l'état de différenciation dans la voie lymphoïde T, elle peut exprimer ou non le marqueur CD34 (perte de CD34 au cours de la différenciation).

Parmi les « progéniteurs de cellules T », on cite les cellules que l'on peut trouver dans le thymus post-natal, c'est-à-dire les cellules thymiques progénitrices précoces (*early thymic progenitor,* ETP) (CD34+/CD45RA+/CD7+), les cellules proT1 (CD34+CD45RA+CD7++CD5-CD1a-), les cellules proT2 (CD34+CD45RA+CD7++CD5+CD1a-), et les cellules preT (CD34-CD7++/CD5+CD1a+). Ces cellules sont bien connues dans l'état de la technique. Elles sont notamment citées par Reimann *et al.* (2012, *op. cit*.), et par Awong *et al.* (2009, *op.cit*.).

Par peptide RGDS, on entend désigner tout peptide ou toute protéine contenant le motif RGDS, de telle sorte qu'il puisse se lier à l'intégrine VLA-5 (voir ci-après). Un tel peptide ou une telle protéine peut être testé par sa capacité à supprimer l'interaction de la fibronectine avec cette intégrine VLA-5, par des méthodes largement documentées dans l'art.

Le peptide RGDS se lie à l'intégrine VLA-5 (Very Late Antigen-5), qui est un dimère composé de CD49e (alpha5) et de CD29 (beta1).

Les domaines de liaison à l'héparine sont connus dans l'art et présents dans de nombreuses protéines se liant à l'héparine. Ils se présentent généralement sous la forme XBBXBX and XBBBXXBX (B = acide aminé basique ; X = acide aminé hydropathique ; Cardin et Weintraub, Arterioscler Thromb Vase Biol. 1989;9:21-32, SEQ ID N° 4 et SEQ ID N° 5).

La présence d'un tel domaine de liaison à l'héparine est particulièrement avantageuse lorsque l'on expose également les cellules CD34+ à un vecteur rétroviral dans le but de les transduire, afin d'obtenir des progéniteurs de cellules T exprimant un transgène.

Un peptide CS-1 ou motif CS-1 est un peptide de 25 acides aminés (DELPQLVTLPHPNLHGPEILDVPST, SEQ ID N° 6), qui a été décrit par Wayner et al., 1989, J. Cell Biol. 109: 1321). Ce motif CS-1 se lie au récepteur VLA-4 (Very Late Antigen-4). Cet antigène est une intégrine dimérique, composée de CD49d (alpha 4) et de CD29 (beta 1).

Dans un mode de réalisation particulier, le fragment de fibronectine est présent dans le milieu de culture ou immobilisé sur la paroi inférieure du récipient. La fibronectine est une protéine, qui sous sa forme naturelle, est un dimère de grande taille en forme de v de 100 nm de long et de 460kDa. Les deux monomères sont reliés par deux ponts disulfure à leur extrémité C-terminale. Par « fibronectine », on entend désigner la protéine fibronectine naturelle (c'est-à-dire n'importe quelle isoforme produite par épissage alternatif), mais également un monomère de cette protéine, ou un fragment de cette protéine (mais contenant le peptide RGDS, ainsi que le peptide CS-1 et le site de liaison à l'héparine).

Une fibronectine particulièrement adaptée pour la mise en œuvre du procédé ainsi décrit est la Retronectin^{®}. Cette protéine correspond à un fragment d'une fibronectine humaine (fragment CH-296 ; Kimizuka et al., J Biochem. 1991 Aug;110(2):284-91 ; Chono et al., J Biochem. 2001 Sep;130(3):331-4) et contient les trois domaines fonctionnels préférés dans le cadre de la mise en œuvre du procédé (le domaine C de liaison aux cellules contenant le peptide RGDS, le domaine de liaison à l'héparine et la séquence CS-1). Cette protéine est déjà utilisée pour améliorer la transduction utilisant des vecteurs rétroviraux (Chono *et al., op cit*)*,* en permettant la colocalisation des cellules cibles (liaisons aux intégrines VLA-4 et VLA-5) et des virions (qui se lient au domaine de liaison à l'héparine). Cette protéine est notamment commercialisée par la société Takara Bio Inc. (Shiga, Japon).

Il est surprenant et inattendu que cette protéine puisse également améliorer la différenciation des cellules CD34+ en progéniteurs de cellules T, y compris lorsque l'on souhaite obtenir de tels progéniteurs pour des cellules transduites par un vecteur viral, dans le cadre d'une thérapie génique, ainsi que pour des cellules adultes.

Ainsi que discuté plus haut, mais sans vouloir être lié par cette théorie, cette amélioration dans la différenciation des cellules CD34+ est probablement liée à la présence du motif RGDS qui se lie à l'intégrine VLA-5, présente sur les cellules et/ou du motif CS-1 qui se lie au récepteur VLA-4.

Dans un mode de réalisation particulier, le fragment de fibronectine est immobilisé (c'est-à-dire est lié à un support solide et n'est pas présent de façon libre en solution (même s'il est possible que certains éléments puissent s'y retrouver s'ils se décollent du support). Ce support solide est préférentiellement la paroi inférieure du récipient dans lequel le procédé est mis en œuvre. Toutefois, on peut aussi envisager de lier le fragment de fibronectine à des billes, telles que des billes de polymères ou des billes magnétiques (d'un diamètre généralement compris entre 1µm et 5 µm). La liaison de la protéine ou du peptide sur ces billes peut être covalente ou non. Les méthodes d'attache d'une protéine ou d'un peptide sur une bille sont bien connues dans l'art. On peut aussi introduire ce fragment de fibronectine dans un milieu semi-solide, tel qu'une gélose ou un gel.

Lorsque le fragment de fibronectine est immobilisé sur le support (en particulier la paroi inférieure du récipient dans lequel le procédé est mis en œuvre), cette immobilisation peut aussi être covalente ou non. Dans un mode de réalisation préféré, cette immobilisation est effectuée de façon non covalente en laissant le fragment de fibronectine être absorbé sur le verre ou le plastique composant la paroi inférieure du récipient.

Dans un mode de réalisation particulier, ainsi que vu plus haut, on effectue à la fois la différenciation des cellules CD34+ en progéniteurs de cellules T, et la transduction desdites cellules CD34+ à l'aide d'un vecteur viral afin d'introduire un gène d'intérêt dans ces cellules. Cela signifie que les cellules exposées au ligand de Notch et au fragment de fibronectine sont également exposées à un surnageant viral, pendant au moins une partie de leur temps d'exposition au ligand de Notch et fragment de fibronectine.

De fait, les inventeurs ont pu mettre en évidence que lorsque des cellules préalablement transduites sont exposées au ligand de Notch (notamment DL-4), la production de lymphocytes T *in vivo* est plus lente que lorsque la transduction est effectuée en même temps que l'exposition à DL-4. L'exposition conjointe permet donc d'accélérer la production de cellules T *in vivo* (après transplantation).

Un second avantage de cette exposition conjointe des cellules au surnageant viral et au ligand de Notch (notamment DL-4) est de raccourcir la période de culture (7 jours au lieu de 11 jours).

Il est à noter qu'une exposition conjointe des cellules au surnageant viral et au ligand de Notch (notamment DL-4) sans la présence du fragment de fibronectine permet également d'accélérer la production de progéniteurs de cellules T.

Ainsi, dans un mode de réalisation préféré, on commence par exposer les cellules au ligand de Notch et fragment de fibronectine pendant un certain temps (de préférence supérieur à 4 heures, de façon plus préférée supérieur à 6 h, voire supérieur à 8h ou supérieur à 10h, mais préférentiellement inférieur à 36 heures, de façon plus préférée inférieur à 30 h, de façon plus préférée inférieure à 24 h), en présence de cytokines, ce qui permet aux cellules d'entrer en cycle de division (préactivation), puis on ajoute le surnageant viral pendant un certain temps (de préférence supérieur à 4 heures, de façon plus préférée supérieur à 6 h, voire supérieur à 8h ou supérieur à 10h, mais préférentiellement inférieur à 30 heures, de façon plus préférée inférieur à 24 h, de façon plus préférée inférieure à 16 h en moyenne).

Si l'on observe que le nombre de cellules transduites est insuffisant, il est possible d'effectuer une seconde transduction, en exposant de nouveau les cellules au vecteur viral selon les modalités décrites ci-dessus.

Ce mode de réalisation est notamment mis en œuvre lorsque l'on souhaite effectuer une greffe autologue de cellules souches hématopoïétiques dans le cadre d'un protocole de thérapie génique. Ainsi, le transgène introduit par transduction avec le surnageant viral est destiné à exprimer une protéine absente ou déficiente chez le patient afin de lui apporter un bénéfice thérapeutique. Parmi les transgènes utilisables (sans que cette liste ne soit limitative ou exhaustive), on peut citer les gènes permettant la thérapie des immunodéficiences (notamment les immunodéficiences combinées sévères, SCID ou non, CID), de HIV, de l'adrénoleucodystrophie liée à l'X, des hémoglobinopathies, notamment de la β-thalassémie ou de l'anémie falciforme. Les immunodéficiences innées ou acquises telles que le SIDA, due au VIH sont donc des maladies pouvant être ciblées par des stratégies de thérapie génique

La transduction est effectuée préférentiellement avec un surnageant rétroviral permettant l'insertion du transgène dans le génome de la cellule. Les vecteurs, notamment des vecteurs lentiviraux, présents dans ledit surnageant sont particulièrement adaptés pour ce type d'application et ont été largement décrits dans la littérature.

Ainsi, le milieu peut aussi contenir un vecteur viral destiné à la transduction desdites cellules CD34+, dans la mise en œuvre du procédé décrit ici. Dans un mode de réalisation particulier, ce vecteur viral est introduit après une période de préactivation des cellules dans le milieu (culture en présence de cytokines permettant aux cellules d'entrer en cycle de division), comprise entre 4 h et 36 h, de préférence entre 6 et 24 h. Le vecteur viral est maintenu dans le milieu au contact des cellules pendant une durée comprise entre 4 h et 30 h, de préférence entre 12 h et 24 h, de façon plus préférée pendant environ 16 h, puis est retiré du milieu. Pour « retirer » le vecteur viral du milieu, les cellules sont récoltées, lavées et remises en culture en présence du ligand de Notch et du fragment de fibronectine.

Dans un mode de réalisation particulier, ledit ligand de Notch est la protéine Delta-like-1 (SEQ ID N° 1) ou un fragment (domaine soluble).

Dans un autre mode de réalisation, le ligand de Notch est la protéine Delta-like-4 (SEQ ID N° 2).

Dans un mode de réalisation particulier, ledit ligand de Notch est une protéine de fusion comprenant le domaine soluble d'un ligand de Notch naturel, fusionné à une région Fc d'une protéine IgG. Le domaine soluble d'un ligand de Notch représente la partie extracellulaire dudit ligand. Celle-ci est bien connue. Ainsi, Varnum-Finney et al. (J Cell Sci. 2000 Dec;113 Pt 23:4313-8) ont décrit une protéine de fusion de la partie soluble de DL-1 avec une portion Fc d'une IGg1. Reimann *et al.* (op. cit) ont décrit une protéine de fusion de la partie soluble de DL-4 (acides aminés 1-526) avec le fragment Fc d'une immunoglobuline IgG2b. Il est ainsi préféré lorsque la protéine IgG est une IgG2.

Le milieu de culture utilisé dans le cadre du présent procédé est tout milieu apte à la culture de cellules CD34+ et de cellules T. On peut notamment citer les milieux α-MEM, DMEM, RPMI 1640, IMDM, BME, McCoy's 5A, StemSpan^{™} (Stem Technologies) ou milieu de Fischer. Un milieu de culture parfaitement adapté et préféré pour la mise en œuvre du procédé décrit dans la présente est le milieu X-VIVO^{™} 20 (Lonza, Bâle, Suisse). Ce milieu a été utilisé notamment par Jonuleit et al. (Eur J Immunol, 1997, 27, 12, 3135-42) et Luft et al. (J Immunol, 1998, 161, 4, 1947-53).

De préférence, on utilise un milieu basal (c'est-à-dire qui permet la croissance des cellules sans qu'il soit nécessaire d'y ajouter des compléments), que l'on va toutefois supplémenter avec sérum, ainsi que des facteurs de croissance et des cytokines.

Ainsi, on ajoute préférentiellement du sérum bovin fœtal (FBS) ou du sérum de veau fœtal (FCS), du sérum humain autologue ou du sérum humain AB dans le milieu de culture basal. De préférence, on complémente ce milieu avec au moins 15 % de sérum fœtal, de façon plus préférée au moins 20 %. Le FBS est particulièrement adapté pour la mise en œuvre de ce procédé. En particulier, on préfère utiliser du FBS défini. Le FBS défini est un sérum de haute qualité qui a été analysé et filtré pour éviter la présence de particules virales. Il est vendu comme tel par de nombreux fournisseurs, comme le HyClone^{™} Defined Fetal Bovine Serum (FBS) de Thermo Scientific^{™}.

Le milieu de culture est également préférentiellement complémenté avec des cytokines et facteurs de croissance. Ces cytokines et facteurs de croissance sont notamment choisis dans le groupe consistant en SCF (Facteur de cellules souches), thrombopoïétine (TPO, également appelée facteur de croissance et de développement des mégacaryocytes, MGDF), Flt3-Ligand (qui est un facteur de croissance hématopoïétique), l'interleukine 3 (IL-3), l'interleukine 7 (IL-7) et le SCF (Facteur de cellules souches). Dans un mode de réalisation particulier, le milieu de culture contient au moins trois, préférentiellement au moins quatre de ces cytokines ou facteurs de croissance.

Dans un mode de réalisation préférée, et notamment pour la génération de précurseurs de lymphocytes T non transduits par un vecteur viral, on ajoute au moins ou exactement trois cytokines.

De préférence, ces trois cytokines sont l'Interleukine-7 (IL-7), le SCF (Facteur de cellules souches) et le Flt-3 Ligand (facteur de croissance hématopoïétique).

Dans un autre mode de réalisation préféré, on ajoute quatre cytokines, c'est-à-dire les trois cytokines mentionnées ci-dessus et le TPO (thrombopoïétine).

Dans un autre mode de réalisation particulier, et notamment pour la génération de précurseurs de lymphocytes T transduits par un vecteur viral, on peut faire varier la nature des cytokines et facteurs de croissance au cours de la mise en œuvre du procédé.

Ainsi, on peut utiliser de l'IL-3, de l'IL-7, du SCF, de la TPO, et du Flt3-L dans le milieu, si l'on réalise l'étape de préactivation des cellules avant ajout du vecteur viral, puis ne supplémenter le milieu qu'avec l'IL-7, le SCF, la TPO, et le Flt3-L, après que l'on a retiré le vecteur.

Les mélanges de cytokines et facteur de croissance mentionnés ci-dessus sont suffisants pour induire la différenciation des cellules CD34+ en précurseurs de lymphocytes T et, généralement, le milieu de culture ne comprend aucun autre cytokine ou facteur de croissance.

Dans le cadre du procédé, la durée totale d'exposition des cellules CD34+ en présence du ligand de Notch et de la protéine ou du peptide présentant le motif RGDS est généralement effectuée pendant une durée préférentiellement supérieure à 3 jours, et inférieure à 10 jours.

Cette exposition peut varier en fonction de la transduction ou non des cellules. Ainsi, une durée d'exposition de trois jours peut s'avérer suffisante pour des cellules souches adultes non-transduites, alors qu'elle sera généralement plus longue en cas de transduction ou de cellules souches infantiles (environ 7 jours).

Les cellules CD34+ sont obtenues à partir d'un échantillon sanguin d'un donneur, d'une ponction de moelle osseuse ou à partir de sang de cordon ombilical. Les méthodes pour trier les cellules CD34+ sont connues dans l'art. On peut notamment utiliser des billes magnétiques présentant un anticorps reconnaissant le CD34+ à leur surface pour ce faire.

De façon préférée, on prépare le récipient de culture cellulaire en immobilisant le ligand de Notch et le fragment de fibronectine sur la surface inférieure, avant d'exposer les cellules CD34+.

La Retronectin^{®} ou une autre fibronectine adhère de façon naturelle au plastique de la boîte de culture cellulaire (boîte de Pétri ou boîte 24 puits, ou autre).

De même, si l'on utilise un ligand de Notch en tant que protéine de fusion avec le fragment Fc d'une immunoglobuline, ce fragment Fc adhère également au plastique.

Il suffit donc de laisser le récipient en présence de ces composés pendant quelques heures afin d'obtenir le revêtement adéquat.

En particulier, le protocole suivant peut être utilisé pour recouvrir la surface inférieure avec le ligand de Notch fusionné avec un fragment Fc, et la Retronectin^{®} :
- préparer une solution contenant le ligand de Notch (5 µg/ml) et la Retronectin^{®} (25 à 50 µg /ml) dans du PBS (Phosphate-Buffered Saline). On peut toutefois aussi utiliser des quantités de Retronectin^{®} aussi basses que 10 à 15 µg /ml sans diminuer les résultats.
- recouvrir les puits d'une plaque de culture cellulaire avec cette solution: 0,5 ml pour une plaque 24 puits ; 1,0 ml pour une plaque 6 puits
- laisser les protéines se déposer pendant 24h à 4°C (voire 48 à 72 heures), ou pendant environ 2 h à 37°C
- laver avec du PBS (à la température de travail)
- utiliser un agent de blocage (PBS + 2% BSA (Albumine de sérum bovin ou humain) ou d'HA (Hémagglutinine humaine) (0,5 ml pour les plaques 24 puits ; 2ml pour les plaques 6 puits) pendant 1 h à 37°C
- laver avec du PBS à 37 °C

Il est clair que la mise au point d'autres concentrations ou d'autres conditions de revêtement est un travail de routine pour l'homme du métier, s'il utilise d'autres protéines ou d'autres récipients de culture cellulaire (flasques, poches... ).

En particulier, les inventeurs ont montré qu'à la concentration de 5 µg/ml, environ 75% de DL-4 se fixe à la surface du récipient. Différentes doses de DL-4 ont été testées et il est préférable que la concentration soit supérieure ou égale à 1,25 µg/ml, une concentration optimale étant entre 2,5 et 5 µg/ml.

Pour ce qui est de la Retronectin^{®}, la concentration de 25 µg/ml est parfaitement adaptée, bien que d'autres concentrations (supérieures ou inférieures) puissent également convenir.

La présente divulgation se rapporte ainsi également à un récipient de culture cellulaire, caractérisée en ce qu'au moins l'une de ses surfaces est recouverte d'un ligand de Notch et d'une protéine ou d'un peptide présentant le motif RGDS et/ou CS-1, et en particulier un fragment de fibronectine tel que décrit ci-dessus.

Le ligand de Notch peut être la partie soluble du ligand naturel de Notch (en particulier DL-1 ou DL-4) et être fusionné avec un fragment Fc d'une immunoglobuline (telle qu'une IgG1 ou une IgG2).

Par exemple, il s'agit de DL-4. Le fragment Fc peut être issu d'une IgG2. Le ligand de Notch immobilisé à la surface de la boîte de culture (et utilisé dans le cadre du procédé décrit plus haut) peut présenter la séquence SEQ ID N° 7.

La protéine présentant le motif RGDS peut être telle que décrite plus haut. Il s'agit préférentiellement d'une fibronectine et en particulier de la Retronectin^{®}.

Ainsi, la boîte de culture cellulaire peut présenter au moins une surface qui a été recouverte d'une protéine de fusion DL-4 / fragment Fc d'une IgG2 et de la Retronectin^{®}.

Les concentrations sont telles que mentionnées ci-dessus, et le récipient a été obtenu, en particulier par la méthode décrite ci-dessus, c'est-à-dire une étape d'addition d'une solution contenant le ligand de Notch, la protéine ou le peptide présentant le motif RGDS et/ou CS-1, en particulier le fragment de fibronectine dans ledit récipient, une étape de repos permettant aux protéines de se déposer et d'adhérer aux parois du récipient, suivies d'étapes de rinçage et préférentiellement de blocage des parois du récipient avec une protéine non-réactive (telle qu'une albumine de sérum).

Dans la mise en œuvre du procédé décrit plus haut, on ajoute les cellules à une concentration comprise entre 10⁶ et 10⁷ cellules CD34+/ml, en particulier de l'ordre de 2×10⁶ cellules CD34+/ml dans le récipient de culture. Suivant la quantité de CD34+ à transduire, on pourra utiliser une boite allant de 2 à 10 cm² (soit respectivement, une plaque allant de 24 puits à 6 puits). De préférence, lorsque l'on utilise une boîte 24 puits, on inocule chaque puits avec entre10⁵ et 10⁶ cellules CD34+ par puits, préférentiellement de l'ordre de 2.10⁵ à 4.10⁵ cellules CD34+ par puits. Dans le cas d'une boîte de culture à 6 puits, on utilise plutôt entre 8.10⁵ et 2.10⁶ cellules par puits.

La quantité de cellules à inoculer peut être adaptée aisément par l'homme du métier en fonction du récipient qu'il utilise.

Les cellules sont placées dans le puits dans le milieu basal choisi, préférentiellement supplémenté avec des cytokines ou facteurs de croissance, ainsi que vu plus haut.

Les concentrations de ces cytokines ou facteurs de croissance sont comprises entre 2 et 300 ng/ml.

De préférence, les cytokines sont ajoutées à une concentration généralement supérieure à 40 ng/l et inférieure à 300 ng/ml ou 200 ng/l, de façon plus préférée à une concentration de l'ordre de 100 ng/ml.

Toutefois, lorsque l'on souhaite générer des progéniteurs de cellules T transduites et que l'on pré-active les cellules avant de les mettre en présence du vecteur viral, on peut utiliser des concentrations plus élevées (de l'ordre de 300-400 ng/ml). Dans ce mode de réalisation, on peut utiliser le SCF et le Flt3-L à des concentrations de l'ordre de 300ng/ml, le TPO et l'IL-7 à des concentrations de l'ordre de 100ng/ml, et l'IL-3 à environ 40ng/ml.

Dans un mode de réalisation préféré, la durée d'exposition des cellules au ligand de Notch et au fragment de fibronectine est préférentiellement supérieure ou égale à 3, voire supérieure ou égale à 5 jours. Elle est généralement inférieure à 10 jours, voire inférieure à 8 jours. Une durée avantageuse est comprise de préférence entre 5 et 8 jours, c'est-à-dire environ 7 jours. Si l'on désire dépasser 7 jours de culture, il est préférable de transférer les cellules dans de nouveaux récipients revêtus du ligand de Notch et du fragment de fibronectine. Cette durée dépend notamment de l'origine des cellules CD34+. Ainsi la durée d'exposition peut être plus courte pour des cellules CD34+ obtenues à partir d'un adulte que pour des cellules obtenues à partir d'un enfant ou de sang de cordon. Ceci est particulièrement étonnant, à la vue de l'art antérieur et de la capacité a *priori* moindre de différenciation de cellules souches adultes. On expose généralement les cellules transduites plus longtemps que les cellules non-transduites.

Après avoir généré les progéniteurs de cellules T, par le procédé décrit ci-dessus, il est possible de purifier ces progéniteurs de cellules T ainsi générés. Cette purification est effectuée par lavage des cellules et suspension dans un milieu basal adapté.

Ces progéniteurs de cellules T peuvent aussi être conditionnés dans une poche afin de pouvoir les injecter à un patient. Dans ce cas, ces cellules sont reconditionnées dans une solution saline contenant du SAH à 5% telle que albunorm^{™} 5% 50 g/L (Octopharma, Lingolsheim, France).

Ces cellules peuvent aussi être congelées selon les méthodes connues dans l'art.

La présente divulgation décrit également des progéniteurs de cellules T pour leur utilisation chez un patient immunodéprimé, en particulier pour permettre une reconstitution immunitaire chez ce patient et/ou l'obtention d'une protection immunitaire vis à vis d'infections chez ledit patient pendant une période de l'ordre de quelques mois (environ au moins deux mois, de préférence environ au moins six mois). Le patient peut être un patient immunodéprimé. Les raisons du déficit peuvent être multiples : déficit immunitaire héréditaire, chimiothérapie pour une leucémie, conditionnement, greffon ne contenant que des cellules souches, traitement post-greffe en prophylaxie de la GVH (réaction du greffon contre l'hôte), âge du receveur, et complications du type infections. Il peut ainsi notamment être immunodéprimé du fait de la déplétion de ses cellules immunitaires suite à la thérapie avant greffe de moelle osseuse. La greffe peut être une allogreffe (dans ce cas, les progéniteurs de cellules T sont préférentiellement issus d'un donneur HLA-partiellement compatible avec le patient), ou une autogreffe (auquel cas les progéniteurs de cellules T ont préférentiellement été transformés par un vecteur afin d'exprimer un gène et/ou une protéine permettant de corriger un défaut génétique chez ledit patient).

Les progéniteurs de cellules T peuvent avoir été obtenus par exposition de cellules CD34+ en présence d'un ligand de Notch (tel que décrit ci-dessus) et d'une protéine ou d'un peptide présentant le motif RGDS et/ou le motif CS1, notamment d'un fragment de fibronectine tel que décrit ci-dessus, dans les conditions mentionnées ci-dessus. L'exposition peut être inférieure ou égale à 10 jours.

Les progéniteurs de cellules T envisagés présentent notamment le marqueur CD7, et peuvent exprimer ou non le marqueur CD34.

La présente divulgation décrit également une méthode de traitement d'un patient immunodéprimé, en particulier en vue de permettre une reconstitution immunitaire, au moins temporaire, chez ce patient, comprenant l'étape d'administrer audit patient des progéniteurs de cellules T, tels que décrits plus tôt. Cette méthode peut également inclure l'étape d'obtention de tels progéniteurs par exposition de cellules CD34+ à un ligand de Notch (tel que décrit ci-dessus) et une protéine ou un peptide présentant le motif RGDS et/ou le motif CS1, notamment un fragment de fibronectine tel que décrit ci-dessus, dans les conditions mentionnées ci-dessus. On administre notamment une quantité thérapeutiquement efficace, c'est-à-dire de l'ordre de 1 à 5×10⁶ /kg progéniteurs, ce qui permet de fournir au patient des cellules capables de jouer un rôle protecteur vis à vis des infections pendant quelques mois (de l'ordre de 6 mois).

Cette administration de progéniteurs de cellules T est effectuée juste avant, juste après ou concomitamment à une greffe de cellules souches hématopoïétiques chez ledit patient. Ainsi que vu plus haut, les cellules injectées peuvent être transformées par un vecteur visant à permettre la correction d'un défaut génétique chez ledit patient.

### Description des Figures

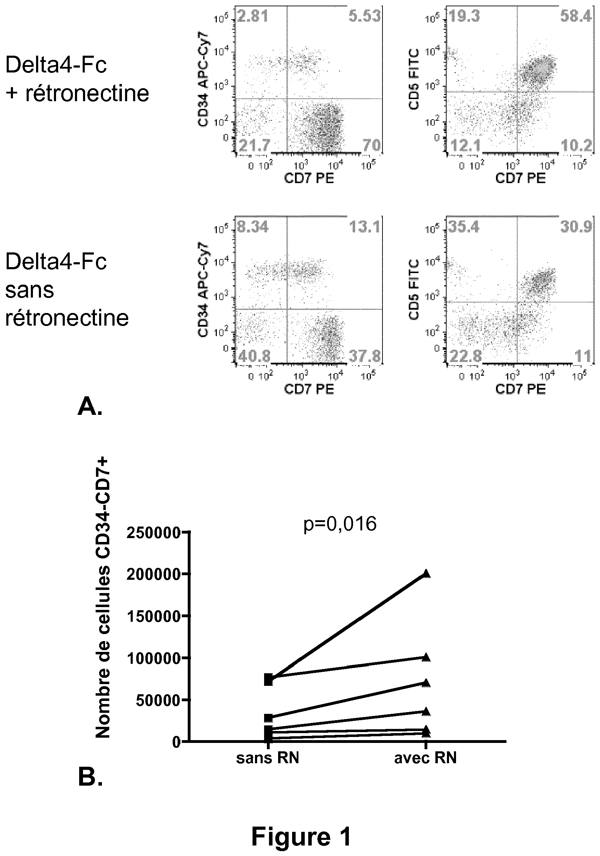

Figure 1 : comparaison du nombre et pourcentage de cellules CD7+CD34+/- obtenus après culture de cellules CD34+ en absence (-RN) ou présence (+RN) de Retronectin^{®} (RN). A. analyse par cytométrie de flux de l'expression des marqueurs CD34, CD7. B. Représentation du nombre de cellules CD34-CD7+ (pour six expériences indépendantes).

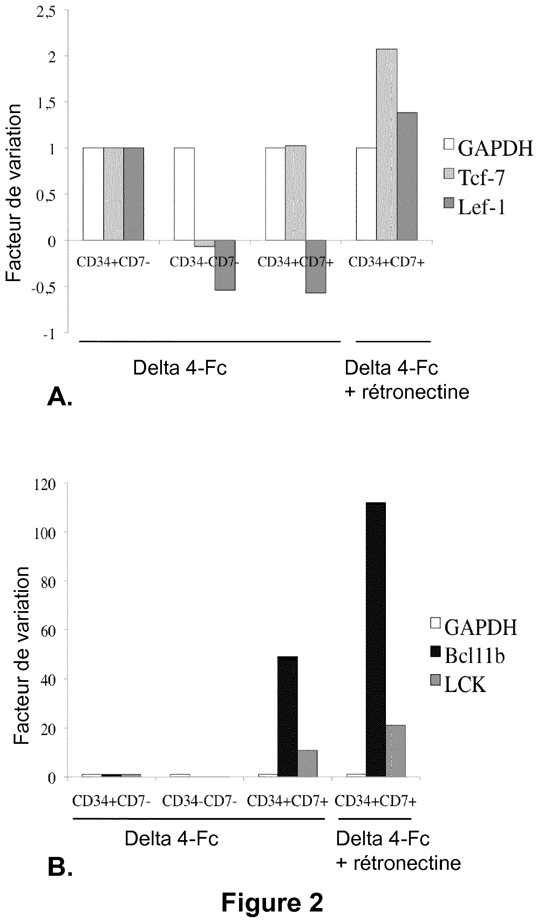

Figure 2 : comparaison de l'expression (par rapport au contrôle GAPDH (glycéraldéhyde-3-phosphate déshydrogénase)) de gènes impliqués dans la différenciation des cellules CD34+ en lymphocytes T. A. gènes Tcf-7 (*transcription factor* 7) et Lef-1 (*lymphoid enhancer-binding factor 1*)*.* B. gènes Bcl11b (*B-cell CLL*/*lymphoma 11B*) et LCK (*lymphocyte-specific protein tyrosine kinase*)*.*

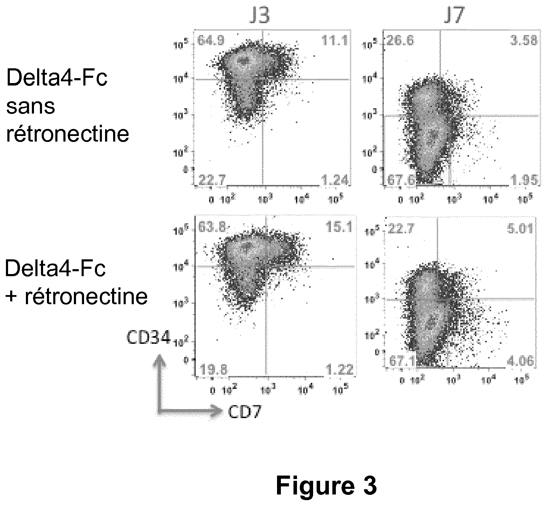

Figure 3 : comparaison du pourcentage de cellules CD7+CD34+/- obtenus après culture en présence ou absence de Retronectin^{®} (analyse par cytométrie de flux de l'expression des marqueurs CD34, CD7), obtenus par culture de cellules CD34+ de sang de donneurs mobilisés.

### Exemples

### Exemple 1 - Matériel et méthodes

a) Préparation des cellules CD34+
   Les cellules CD34+ sont isolées par séparation des cellules mononucléées sur gradient de Ficoll et tri immunomagnétique, ainsi que décrit dans l'art antérieur (Six et al., J Exp Med 2007 204 :3085).
b) Préparation du ligand de Notch soluble
   Le ligand de Notch soluble est une protéine de fusion générée par clonage de la partie soluble du ligand de Notch et de la partie constante d'immunoglobuline humaine, ainsi que décrite dans Reimann *et al.* (2012, *op. cit*.)*.* La séquence de la protéine de fusion est SEQ ID N°7.
c) Préparation des récipients
   i. Sans Retronectin^{®}
      Le récipient est composé d'un puits de culture contenant du milieu de culture, sur lequel le ligand de Notch soluble a été immobilisé selon la technique décrite dans Reimann *et al.* (2012, *op. cit*.).
   ii. Avec Retronectin^{®}
      La Retronectin^{®} est immobilisée en même temps que le ligand de Notch soluble dans le puits de culture. La concentration de Retronectin^{®} est de 25 à 50 µg/ml. On travaille à température ambiante ou à 4°C, sous Postes de sécurité microbiologique (PSM).
d) Exposition des cellules CD34+
   Les cellules CD34+ sont mises en culture dans le récipient à une concentration comprise entre 2×10⁴ et 1,5×10⁵ cellules/ml. La culture dure entre 3 et 10 jours, ainsi que décrit dans Reimann *et al.* (2012, *op. cit*.).
e) Analyse des cellules CD34+
   L'analyse des cellules cultivées comprend : une numération des cellules vivantes, une analyse par cytométrie de flux de l'expression des marqueurs CD34, CD7 et éventuellement CD5 et CD1a et par une coculture secondaire en conditions de dilution limite sur un stroma OP9/DL-1 qui permet de quantifier les précurseurs T générés par la culture (Reimann C Stem Cells 2012, 30(8):1771-80).
Elle est éventuellement complétée par une analyse du profil d'expression génique par RT-PCR quantitative portant sur des molécules importantes pour l'engagement et la différenciation des cellules CD34+ en lymphocytes T (TCF7, GATA3, RAG1, IL7R, BCL11B, LEF1, PTCRA).

### Exemple 2 - Comparaison des résultats obtenus en présence ou non de Retronectin^{®}. en l'absence de transduction des cellules

### 1. Résultats obtenus avec des cellules CD34+ de sang de cordon (6 expériences)

On observe une augmentation du pourcentage et du nombre de cellules CD7+CD34+/- obtenus après culture en présence de Retronectin^{®} (Figure 1).

On observe également une augmentation de la fréquence de précurseurs T présente dans la culture à J7 et mesurée en dilution limite par coculture sur OP9/DL-1 (Tableau I).

**TableauI**

| Précurseurs T à J7 (2×10⁴ CD34+ à J0) | Fréquence | Nombre |
|---|---|---|
| En absence de Retronectin^{®} | 1/30 | 733 |
| En présence de Retronectin^{®} | 1/21 | 2108 |

On peut également observer une augmentation de l'expression (par rapport au gène endogène GAPDH (glycéraldéhyde-3-phosphate déshydrogénase)) de gènes impliqués dans la différenciation des cellules CD34+ en lymphocytes T (Figure 2) 2. Résultats obtenus avec des cellules CD34+ de sang de donneurs mobilisées

On observe une augmentation du pourcentage de cellules CD7+CD34+/- après culture en présence de Retronectin^{®}par rapport à ce que l'on obtient après culture en absence de Retronectin^{®} (Figure 3).

On observe également une augmentation de la fréquence de précurseurs T présente dans la culture à J7 et mesurée en dilution limite par coculture sur OP9/DL-1 (Tableaux II et III)

### Résultats obtenus à partir de 10⁵ CD34+

**Tableau II**

| Exp1 | Absence de Retronectin^{®} | | Présence de Retronectin^{®} | |
|---|---|---|---|---|
| | Fréquence | Total proqéniteurs | Fréquence | Total proqéniteurs |
| J3 | 1/42 | 2133 | 1/33 | 3240 |
| J7 | 1/100 | 1600 | 1/46 | 2613 |

**Tableau III**

| Exp2 | Absence de Retronectin^{®} | | Présence de Retronectin^{®} | |
|---|---|---|---|---|
| | Fréquence | Total proqéniteurs | Fréquence | Total proqéniteurs |
| J3 | 1/19 | 4109 | 1/10 | 8836 |
| J7 | 1/72 | 1654 | 1/19 | 6709 |

### 3. Conclusion

Ces résultats montrent bien que la présence conjointe de Retronectin^{®}et du ligand de DL-4 permet d'améliorer la génération de précurseurs engagés dans la voie de différenciation lymphoïde T (progéniteurs de cellules T) à partir de cellules souches CD34+.

### Exemple 3 - Résultats obtenus en présence de Retronectin^{®}, pour des cellules transduites

Les cellules CD34+ de moelle osseuse de patient sont préactivées pendant 24h à la concentration de 2×10⁶ cellules/dans un milieu X-VIVO20 contenant SCF 300ng/ml, Flt3-L 300ng/ml, TPO 100ng/ml, IL-3 40ng/ml, IL-7 100ng/ml et 20% de sérum sur un substrat obtenu après avoir recouvert les plaques de culture d'une solution de Retronectin^{®} 50µg/ml et de DL4/Fc 5 µg/ml.

Entre J1 et J2, les cellules sont soumises à une transduction à l'aide du vecteur viral dans le milieu de pré-activation enrichi de protamine sulfate à 4 µg/ml.

A J3, les cellules sont récoltées, lavées et remises en culture jusqu'à J7 sur un substrat protéique DL4/Fc dans un milieu supplémenté en sérum (20%), SCF (100ng/ml), Flt3-L (100ng/ml), TPO (100ng/ml) et IL-7 (100ng/ml).

Ceci permet aux progéniteurs CD34+ d'entrer dans la voie de différenciation T, ce qui est attesté par l'apparition d'un grand nombre de cellules portant le marqueur CD7.

La preuve de cet engagement est confirmée par la production considérablement accélérée de lymphocytes T.

Les expériences *in vitro* ont révélé, qu'à la fin de la transduction des CSH CD34+, la culture en présence de DL4/Fc et du fragment de Retronectin^{®} contient des progéniteurs lymphoïdes T CD34+/-CD7+ (14%). On observe également l'apparition de lymphocytes T double positifs CD4+CD8+ (8%) et de lymphocytes T CD3+ (8%) dès J25, alors que le protocole classique de transduction seul (sans exposition au DL4/Fc + Retronectin^{®}) ne permet d'obtenir que moins de 1% de lymphocytes T (CD4+CD8+ ou CD3+) à J25.

*In vivo,* la greffe des progéniteurs générés selon le protocole défini, à des souris NOD/SCID/γc ko montre l'induction d'une thymopoïèse humaine, environ 6 semaines après la greffe de cellules transduites et exposées à DL-4/Fc + Retronectin^{®}.

Aucune thymopoïèse n'est observée dans les receveurs de cellules transduites dans les conditions classiques.

## Revendications

1. Procédé *in vitro* de génération de progéniteurs de cellules T, comprenant l'étape d'exposer des cellules CD34+ dans un milieu en présence d'un ligand de Notch, immobilisé sur un support, ledit ligand de Notch étant le domaine soluble du ligand Delta-like-4, fusionné à une région Fc d'une protéine IgG, et en présence d'un fragment d'une fibronectine, ledit fragment comprenant les motifs RGDS, CS-1 et un domaine de liaison à l'héparine, dans lequel le temps d'exposition des cellules CD34+ audit ligand de Notch en présence dudit fragment d'une fibronectine est inférieur à 10 jours, de préférence compris entre 3 et 8 jours.

2. Procédé selon la revendication **1, caractérisé en ce que** lesdites cellules CD34+ ont été isolées à partir d'un patient adulte.

3. Procédé selon l'une des revendications **1** à **2, caractérisé en ce que** la durée d'exposition des cellules au ligand de Notch et au fragment de fibronectine est comprise entre 3 et 8 jours.

4. Procédé selon l'une des revendications **1** à **3, caractérisé en ce que** ladite protéine IgG est une IgG2.

5. Procédé selon l'une des revendications **1** à **4, caractérisé en ce que** ledit fragment de fibronectine est le fragment CH-296 de fibronectine humaine.

6. Procédé selon l'une des revendications **1** à **5, caractérisé en ce que** ledit fragment de fibronectine est immobilisé sur la surface inférieure du récipient de culture.

7. Procédé selon l'une des revendications **1** à **6, caractérisé en ce que** ledit milieu contient également un vecteur viral destiné à la transfection desdites cellules CD34+, pendant au moins une partie du temps d'exposition desdites cellules CD34+ audit ligand de Notch et audit fragment de fibronectine.

8. Procédé selon l'une des revendications **1** à **7, caractérisé en ce que** le milieu contient au moins 15 % de FBS, de préférence au moins 20 % de FBS.

9. Procédé selon l'une des revendications **1** à **8, caractérisé en ce que** le milieu comprend au moins trois cytokines ou facteurs de croissance choisies dans le groupe consistant en l'Interleukine 7 (IL-7), le SCF (Facteur de cellules souches), la thrombopoïétine (TPO), et le ligand Flt3 (FLT3L).

10. Procédé selon l'une des revendications **1** à **9, caractérisé en ce que** le milieu comprend de l'Interleukine 7, du SCF, de la thrombopoïétine, et du ligand Flt3.

11. Procédé selon la revendication **10, caractérisé en ce que** le milieu ne comprend aucune autre cytokine ou facteur de croissance.

12. Procédé de préparation de progéniteurs de cellules T, comprenant les étapes de mise en oeuvre du procédé selon l'une des revendications **1** à **11,** et de purification des progéniteurs de cellules T générés.

13. Procédé selon la revendication **12, caractérisé en ce qu'**il comprend également une étape de conditionnement desdits progéniteurs de cellules T dans une poche pour injection à un patient.

## Patentansprüche

1. In-vitro-Verfahren zur Erzeugung von T-Zell-Vorläufern, umfassend den Schritt des Exponierens von CD34+-Zellen in einem Medium in Gegenwart eines Notch-Liganden, der auf einem Träger immobilisiert ist, wobei der Notch-Ligand die lösliche Domäne des Delta-ähnlichen Liganden 4 ist, die an eine Fc-Region eines IgG-Proteins fusioniert ist, und in Gegenwart eines Fragments eines Fibronektins, wobei das Fragment das RGDS-Motiv, das CS-1-Motiv und eine Domäne zur Verbindung mit Heparin umfasst, wobei die Expositionszeit von CD34+-Zellen mit dem Notch-Liganden in Gegenwart des Fragments eines Fibronektins weniger als 10 Tage beträgt, vorzugsweise zwischen 3 und 8 Tagen liegt.

2. Verfahren nach Anspruch **1, dadurch gekennzeichnet, dass** die CD34+-Zellen von einem erwachsenen Patienten isoliert wurden.

3. Verfahren nach einem der Ansprüche **1** bis **2, dadurch gekennzeichnet, dass** die Expositionsdauer von Zellen mit dem Notch-Liganden und mit dem Fibronektinfragment zwischen 3 und 8 Tagen liegt.

4. Verfahren nach einem der Ansprüche **1** bis **3, dadurch gekennzeichnet, dass** das IgG-Protein ein IgG2 ist.

5. Verfahren nach einem der Ansprüche **1** bis **4, dadurch gekennzeichnet, dass** das Fibronektinfragment das CH-296-Fragment von humanem Fibronektin ist.

6. Verfahren nach einem der Ansprüche **1** bis **5, dadurch gekennzeichnet, dass** das Fibronektinfragment auf der unteren Oberfläche des Kulturbehälters immobilisiert ist.

7. Verfahren nach einem der Ansprüche **1** bis **6, dadurch gekennzeichnet, dass** das Medium außerdem einen viralen Vektor, der zur Transfektion der CD34+-Zellen vorgesehen ist, während mindestens einem Teil der Expositionszeit der CD34+-Zellen mit dem Notch-Liganden und mit dem Fibronektinfragment enthält.

8. Verfahren nach einem der Ansprüche **1** bis **7, dadurch gekennzeichnet, dass** das Medium mindestens 15% FBS, vorzugsweise mindestens 20% FBS enthält.

9. Verfahren nach einem der Ansprüche **1** bis **8, dadurch gekennzeichnet, dass** das Medium mindestens drei Zytokine oder Wachstumsfaktoren umfasst, die aus der Gruppe bestehend aus Interleukin 7 (IL-7), SCF (Stammzellfaktor), Thrombopoetin (TPO) und Flt3-Ligand (FLT3L) ausgewählt sind.

10. Verfahren nach einem der Ansprüche **1** bis **9, dadurch gekennzeichnet, dass** das Medium Interleukin 7, SCF, Thrombopoetin und Flt3-Ligand umfasst.

11. Verfahren nach Anspruch **10, dadurch gekennzeichnet, dass** das Medium kein anderes Zytokin oder keinen anderen Wachstumsfaktor umfasst.

12. Verfahren zur Herstellung von T-Zell-Vorläufern, umfassend die Schritte des Durchführens des Verfahrens nach einem der Ansprüche **1** bis **11** und des Aufreinigens von erzeugten T-Zell-Vorläufern.

13. Verfahren nach Anspruch **12, dadurch gekennzeichnet, dass** es außerdem einen Schritt des Verpackens der T-Zell-Vorläufer in einem Beutel zur Injektion an einen Patienten umfasst.

## Claims

1. An *in vitro* method for generating T cell progenitors, comprising the step of exposing CD34+ cells in a medium in the presence of a Notch ligand immobilized on a support, said Notch ligand being the soluble domain of the Delta-like-4 ligand, fused to a Fc region of an IgG protein, and in the presence of a fragment of a fibronectin, said fragment comprising the RGDS and CS-1 patterns and a heparin-binding domain, wherein the exposure time of CD34+ cells to said Notch ligand in the presence of said fragment of a fibronectin is of less than 10 days, preferably is comprised between 3 and 8 days.

2. The method according to claim **1**, wherein said CD34+ cells have been isolated from an adult patient.

3. The method according to claim **1** or claim **2**, wherein the exposure time of the cells to the Notch ligand and the fibronectin fragment is comprised between 3 and 8 days.

4. The method according to one of claims **1** to **3**, wherein said IgG protein is an IgG2.

5. The method according to one of claims **1** to **4**, wherein said fibronectin fragment is the CH-296 fragment of human fibronectin.

6. The method according to one of claims **1** to **5**, wherein said fibronectin fragment is immobilized on the lower surface of a culture vessel.

7. The method according to one of claims **1** to **6**, wherein said medium also contains a viral vector intended for the transfection of said CD34+ cells, during at least one part of the exposure time of said CD34+ cells to said Notch ligand and said fibronectin fragment.

8. The method according to one of claims **1** to **7**, wherein the medium comprises at least 15% of FBS, preferably at least 20% of FBS.

9. The method according to one of claims **1** to **8**, wherein the medium comprises at least three cytokines or growth factors selected from the group consisting of Interleukin-7 (IL-7), SCF (Stem cell Factor), thrombopoietin (TPO) and Flt3 ligand (FLT3L).

10. The method according to one of claims **1** to **9**, wherein the medium comprises Interleukin-7, SCF, thrombopoietin and FLT3 ligand.

11. The method according to claim **10**, wherein the medium does not comprise any other cytokine or growth factor.

12. A method for preparing T cell progenitors, comprising the steps of implementing the method according to one of claims **1** to **11**, and of purifying the T cell progenitors generated.

13. The method according to claim **12**, wherein the method further comprises a step of conditioning said T cell progenitors in a pouch for injection to a patient.
